# EUROPEAN PATENT APPLICATION

(11) **EP 1 938 794 A2**
(43) Date of publication of application: **02.07.2008**
(21) Application number: 07019175.4
(22) Date of filing: 28.09.2007
(51) Int. Cl.: A61K 8/60, A61K 8/64, A61K 8/98, A61K 8/99, A61Q 19/00, A61Q 19/08

(54) **Compounding ingredients for cosmetic formulation for improving skinditch density and cosmetics**

(30) Priority: 21.12.2006 JP 2006344971
(71) Applicant: Nissei Bio Co., Ltd., Erniwa-shi Hokkaido 061-1374 (JP)
(72) Inventor: Matsunaga, Masaji, Tokyo (JP); Yoshida, Fumito, Tokyo (JP); Mori, Takao, Sapporo-shi Hokkaido (JP); Sugi, Masahito, Eniwa-shi Hokkaido (JP); Hasegawa, Eishi, Eniwa-shi Hokkaido (JP)
(74) Representative: HOFFMANN EITLE

(57) **Abstract**

It is intended to provide compounding ingredients for cosmetics for improving skinditch density and cosmetics. The present invention provides compounding ingredients for cosmetics containing, as an active ingredient, nucleoprotein and/or DNA or RNA enzymatic or hydrolytic degradation product(s), a deoxyoligonucleotide, deoxymononucleotide, oligopeptide, oligonucleotide, or mononucleotide separated from the degradation product, or a mixture of at least two members selected from the degradation products and the compounds, and cosmetics containing the compounding ingredients for costmetics. The active ingredients such as the deoxyoligonucleotide have a relatively small molecular weight and as such, are easily transdermally absorbed. The transdermally absorbed active ingredients promote skin cellular regeneration or activation. Thus, these active ingredients, when applied to the epidermis in the face, produce the effect of improving skinditch density (texture), the effect of improving the water retention ability or sebum level balance of the skin, and the effect of preventing wrinkles and the like.

## Description

### BACKGROUND OF THE INVENTION

### Field of the Invention

The present invention relates to compounding ingredients for cosmetics for promoting human skin cellular regeneration or activation and improving skinditch density (texture) and to cosmetics containing the compounding ingredients.

### Description of the Related Art

Skin roughness and spots have been thought to be caused by various matters such as the influence of hormone balances attributed to aging, the stimulation of ultraviolet radiation from sunlight, the harmful effects of increased peroxide concentrations in the body on the epidermis, sebum overproduction, reduction in the level of blood flow into the epidermis, malnutrition, and mental stresses.

Facial skin roughness and wrinkles are of serious aesthetic concern to people. This is obvious from an increase in the number of cosmetic products aimed not only at women but at men in recent years. The face is a major factor that makes a first impression to others. Therefore, many people, irrespective of age and sex, long for shiny and firm skin (beautiful skin). Hence, public interest has been raised increasingly in cosmetics for improving skin roughness and wrinkles.

Many attempts have been made on conventional cosmetics to enhance skin moisturizing effects and make the skin shiny by adding thereto moisturizing agents such as glycerin or oil ingredients. Recently, cosmetics intended to make the skin beautiful have emerged, which comprise L-ascorbic acid and derivatives thereof that work to suppress melanin production and promote collagen production.

In reflection of growing public interest in health, health foods have been provided in recent years, which use deoxyribonucleic acid (DNA), ribonucleic acid (RNA), or a nucleoprotein as a raw material or active ingredient. It has also been proposed to reduce the molecular weight of a high-molecular-weight nucleoprotein for making the nucleoprotein water-soluble or easily digestible (Japanese Patent Laid-Open No. 2004-16143).

Deoxyribonucleic acid (DNA), ribonucleic acid (RNA), or a nucleoprotein has been known to have anti-aging effects. However, sufficient attempts have not been made to apply these components to cosmetics.

Skin troubles such as skin roughness and wrinkles are caused by various factors such as facial skin aging, poor blood circulation, the influence of ultraviolet radiation, and stresses. The direct cause of these skin troubles is cellular regeneration function impaired due to reduction in skin cell metabolism.

No conventional cosmetics exerted their functions so as to act on the mechanism of skin cell metabolism.
These conventional cosmetics merely pursued effects on skin surface. Therefore, they do not have sufficient effects on skin roughness and wrinkles.

Moreover, the approaches to applying L-ascorbic acid and derivatives thereof to cosmetics are not stable and do not sufficiently provide the effect of preventing inflammation induced by ultraviolet radiation. Therefore, it is difficult to directly seek ingredients containing these compounds as active ingredients in cosmetics.

Specifically, products have been desired, which promote skin cellular regeneration by enhancing human skin cellular regeneration and activation abilities, prevent skin roughness and wrinkles, and make the skin highly beautiful. However, these effects of conventional cosmetics are still insufficient.

Hence, it has been desired to develop novel cosmetics for activating cells themselves and making the skin vital.

The present inventors have conducted diligent studies to obtain novel cosmetics for improving human skin texture and have consequently completed the present invention by finding out that a degradation product comprising a deoxyoligonucleotide, deoxymononucleotide, or oligopeptide obtained from a nucleoprotein and/or DNA by enzymatic degradation or hydrolysis treatment, a decomposition product comprising an oligonucleotide or mononucleotide obtained from RNA by enzymatic degradation or hydrolysis treatment, or a mixture thereof has excellent cellular regeneration and activation effects, thereby leading to the effect of improving skinditch density, the effect of improving the water retention ability or sebum level balance of the skin, and the effect of preventing wrinkles and the like.

### SUMMARY OF THE INVENTION

Specifically, compounding ingredients for cosmetics according to the present invention is
compounding ingredients for cosmetics for improving skinditch density, comprising:
a degradation product comprising 20 to 50% fractions with a molecular weight of 1000 to 3000 obtained from a nucleoprotein and/or DNA by enzymatic degradation or hydrolysis treatment for reducing the molecular weight, or a deoxyoligonucleotide, deoxymononucleotide, or oligopeptide separated from the degradation product;
a degradation product comprising 20 to 50% fractions with a molecular weight of 1000 to 3000 obtained from RNA by enzymatic degradation or hydrolysis treatment for reducing the molecular weight, or an oligonucleotide or mononucleotide separated from the degradation product; or
a mixture of at least two members selected from the nucleoprotein and/or DNA degradation product(s), the RNA degradation product, the deoxyoligonucleotide, the deoxymononucleotide, the oligopeptide, the oligonucleotide, and the mononucleotide.

In a preferable aspect of the compounding ingredients for cosmetics of the present invention, the nucleoprotein and the DNA are obtained from milt of fishes such as salmons, trouts, herrings, and cods.

The RNA is obtained from yeast, preferably, yeast selected from the group consisting of beer yeast, torula yeast, milk yeast, and baker's yeast.

Cosmetics according to the present invention comprises the compounding ingredients for costmetics for improving skinditch density.

The compounding ingredients for cosmetics of the present invention comprises, as an active ingredient, a degradation product comprising 20 to 50% fractions with a molecular weight of 1000 to 3000 obtained from a nucleoprotein and/or DNA or from RNA by enzymatic degradation or hydrolysis treatment for reducing the molecular weight, a deoxyoligonucleotide, deoxymononucleotide, oligopeptide, oligonucleotide, or mononucleotide separated from the degradation product, or a mixture of at least two members selected from the degradation products and the compounds.

The active ingredients such as the deoxyoligonucleotide have a relatively small molecular weight and as such, are easily transdermally absorbed. The transdermally absorbed active ingredients work to promote skin cellular regeneration or activation ability. Thus, these active ingredients, when applied to the epidermis in the face, produce the effect of improving skinditch density (texture), the effect of improving moisture and sebum levels, and the effect of improving wrinkles and make the skin beautiful.

Moreover, the cosmetics of the present invention contain the compounding ingredients. Therefore, the cosmetics, when applied to the epidermis in the face, exhibits the excellent effect of preventing skin roughness and wrinkles possessed by the compounding ingredients for cosmetics and makes the skin beautiful.

### BRIEF DESCRIPTION OF THE DRAWINGS

FIG. 1 is a diagram showing an HPLC analysis example of a oligonucleotide in a nuclease-treated DNA product (degradation product);
FIG. 2 is a photographed image showing changes in the skin states of subjects in their thirties before start of a test and after 4 weeks of start of the test;
FIG. 3 is a photographed image showing changes in the skin states of subjects in their fifties before start of a test and after 4 weeks of start of the test;
FIG. 4 is a diagram showing test results of moisture and sebum levels and an average value of each age bracket;
FIG. 5 is a diagram showing test results of skinditch density and wrinkles and an average value of each age bracket;
FIG. 6 is a diagram showing an HPLC analysis example of a deoxyoligonucleotide in a nuclease-treated nucleoprotein product (degradation product); and
FIG. 7 is a diagram showing an HPLC analysis example of an oligonucleotide in a nuclease-treated RNA product (degradation product).

### DETAILED DESCRIPTION OF THE PREFERRED EMBODIMENTS

Active ingredients in compounding ingredients for cosmetics of the present invention, which are also used as purified products, are obtained as follows: a deoxyoligonucleotide or deoxymononucleotide can be obtained from DNA by enzymatic degradation or hydrolysis treatment; an oligonucleotide or mononucleotide can be obtained from RNA by enzymatic degradation or hydrolysis treatment; and an oligopeptide can be obtained from a nucleoprotein by enzymatic degradation or hydrolysis treatment.

The DNA and the nucleoprotein can be obtained by extraction and purification from, for example, milt of fishes . The fishes are, for example, salmons, trouts, herrings, and cods and are, particularly preferably, salmons.

Hereinafter, the DNA will be described in more detail.

The DNA as a raw material for producing the compounding ingredients for cosmetics of the present invention may be in various forms and may be, for example, double-stranded, single-stranded, or circular DNA. Supply sources of the DNA are various organisms such as animals, plants, and microorganisms. The testes (milt) of fishes, particularly, salmons, trout, herrings, and cods, which are obtained as fish processing wastes, are especially rich in DNA. Nevertheless, they have not been utilized effectively as resources so far, and most of them have been discarded. Hence, the utilization of the DNA derived from these testes is also desirable from the viewpoint of the recycling of wastes. Alternatively, DNA obtained from the thymus of mammals or birds, for example, cattle, pigs, and chickens, can be used. Furthermore, synthetic DNA can also be used.

To obtain DNA from milt of fishes, extraction and purification methods described in Japanese Patent Application Laid-open No. 2005-245394 can be used.

Specifically, milt of fishes is first crushed. The crushed milt of fishes is subjected to proteolytic enzyme (protease) treatment under conditions that do not degrade DNA. The enzymatically treated solution is filtered. Then, the filtrate is subjected to dialysis treatment using a hollow fiber membrane with a molecular weight cut-off of 2000 to 1000000. Degraded proteins and ions are removed, while double-stranded DNA is concentrated. Furthermore, the double-stranded DNA salt is precipitated from the dialyzed solution, or the solution is concentrated. These precipitates or concentrates are collected.

The DNA salt obtained by this method is dried, and these dried DNA salt powders can be used as a raw material for producing the compounding ingredients for cosmetics of the present invention.

The RNA can be obtained by extraction and purification from yeast selected from the group consisting of beer yeast, torula yeast, milk yeast, and baker's yeast.

An enzyme for treating the DNA and the RNA is, for example, nuclease and is, particularly preferably, nuclease derived from blue mold.

The oligopeptide is obtained by hydrolysis with protease and nuclease from the nucleoprotein contained in milt of fishes.

The protease is mainly composed of trypsin. Trypsin is serine protease with high specificity, which selectively hydrolyzes peptide bonds on the carboxyl side of arginine and lysine. Therefore, this serine protease is suitable to hydrolysis of protamine rich in arginine. The protease can also include, in addition to trypsin, other proteases, for example, chymotrypsin. Examples of favorable protease can include protease manufactured by Novozymes Japan Ltd.

The nuclease hydrolyzes 3',5'-phosphodiester bonds of deoxyribonucleic acid (DNA) and ribonucleic acid (RNA) and produces 5'-(deoxy)nucleotides in oligo forms by depolymerization. The properties of the nuclease are not particularly limited. It is preferred that the nuclease should possess thermal stability to some extent. Such nuclease is commercially available from, for example, Amano Enzyme Inc. and Sigma.

A reaction temperature is important for the hydrolysis treatment of the DNA, the RNA, and the nucleoprotein with the nuclease. The reaction temperature must fall within a range of 60 to 75°C and is most preferably 70°C. At a reaction temperature lower than the temperature range, the molecular weights of the DNA, the RNA, and the nucleoprotein are not sufficiently reduced, and their degradation products are not rendered water-soluble. On the other hand, at a reaction temperature higher than the temperature range, their molecular weights are excessively reduced, and the excellent effects of the nucleoprotein might be lost.

Thus, the hydrolysis treatment of the DNA, the RNA, and the nucleoprotein with the nuclease at 60 to 75°C can reduce their molecular weights so that the degradation products comprise 20 to 50% fractions with a molecular weight of 1000 to 3000 and can usually comprise fractions with a molecular weight of 1000 or lower in an amount larger than that of the fractions with a molecular weight of 1000 to 3000, for example, 30 to 50% fractions with a molecular weight of 1000 or lower. As a result, DNA, RNA, and nucleoprotein degradation products having both water solubility and transdermal absorbability can be produced.

The nucleoprotein and DNA degradation products obtained by the treatment comprise a deoxyoligonucleotide, a deoxymononucleotide, and an oligopeptide with a reduced molecular weight in a major or large portion and comprise a deoxynucleotide and the like with an insufficiently reduced molecular weight in a very small portion.

Alternatively, the RNA degradation product obtained by similar treatment comprises an oligonucleotide and a mononucleotide in a major or large portion and comprises a nucleotide and the like with an insufficiently reduced molecular weight in a very small portion.

Thus, almost all or most of the nucleotides (deoxyoligonucleotide, deoxymononucleotide, oligonucleotide, and mononucleotide) contained in these degradation products are composed of mono forms that do not originally assume a helix, completely single-stranded oligo forms, and oligo forms that have a double helix structure in only a portion thereof. In other words, the proportion of a double helix in the nucleotides contained in the degradation products does not exceed 20% even if the molecular weights are not sufficiently reduced as described above.

The hydrolysis treatment of the nucleoprotein is protease treatment for obtaining the oligopeptide or nuclease treatment for obtaining the deoxyoligonucleotide. Preferably, the protease treatment is first performed, followed by the nuclease treatment. This is desirable from the viewpoint of operational convenience and the quality of the obtained final products.

The degradation product obtained from a nucleoprotein and/or DNA or from RNA by enzymatic degradation or hydrolysis treatment can be used directly (without purification) as an active ingredient in the compounding ingredients for cosmetics of the present invention. The degradation product may contain, for example, amino acids.

Compounds separated and purified from the nucleoprotein and/or DNA or RNA degradation product(s) by routine separation and/or purification means, that is, a deoxyoligonucleotide, a deoxymononucleotide, an oligopeptide, an oligonucleotide, and a mononucleotide, can be used as an active ingredient in the compounding ingredients for cosmetics of the present invention.

In this context, it is preferred that the deoxyoligonucleotide and the oligonucleotide contained in the nucleoprotein, DNA, and RNA degradation products or separated and purified from the degradation products by routine separation and/or purification means should be 2 to 12 in chain length.

The degradation products and the compounds may be used alone or may be used as a mixture of at least two of them.

When the degradation products and the compounds are mixed for use, a mixing ratio thereof may be selected appropriately.

In this context, it is particularly preferred that the degradation products and the compounds should contain at least one of the deoxyoligonucleotide and the oligonucleotide of 2 to 12 in chain length and has a total content of the deoxyoligonucleotide and the oligonucleotide of 2 to 12 in chain length of 20% or more with respect to the total amount of the degradation products and the compounds.

The concentration of the active ingredient (the degradation products and/or the compounds) in the compounding ingredients for cosmetics of the present invention may be selected appropriately.

The degradation products or the compounds may be used in combination with additional routine additive ingredients for compounding ingredients for cosmetics at a predetermined ratio.

Moreover, cosmetics of the present invention comprises by containing the cosmetic ingredients for cosmetics.

A dosage form that may be assumed by the cosmetics of the present invention may be any appropriately selected dosage form applicable to the skin. Preferably, the cosmetics assume a form directly applied to the skin, such as lotions, milky lotions, creams, gels, jellies, essences, packs, masks, and foundations. Moreover, these cosmetics can be used not only in the facial skin but in the whole body such as the neck and the limb.

In the cosmetics of the present invention, ingredients known in the art, which may be formulated in existing cosmetics, can be formulated in addition to the compounding ingredients for cosmetics. For example, perfumes and moisturizing agents can be formulated alone or in combination.

In the cosmetics of the present invention, vitamin E or derivatives thereof (e.g., vitamin E acetate), vasodilators (e.g., acetylcholine derivatives), skin function promoters (e.g., cepharanthine), glycyrrhetinic acid or derivatives thereof, female hormone drugs (e.g., estradiol and estrone), amino acids (e.g., serine, methionine, and arginine), and vitamins (e.g., vitamin A, vitamin B₁, vitamin B₆, biotin, pantothenic acid, and derivatives thereof) can also be formulated alone or in combination as drug ingredients.

In the cosmetics of the present invention, additives usually used in skin external preparations such as cosmetics or pharmaceutical drugs, for example, oils, preservatives, surfactants, dispersion stabilizers, thickeners, humectants, UV absorbents, antioxidants, pH adjusters, purified water, and alcohols can further be formulated alone or in combination, if necessary.

### [Examples]

The present invention will be described specifically and in more detail with reference to Examples and Comparative Examples shown below. However, Examples below are provided only for illustrative purposes for the present invention and do not limit the technical scope of the present invention.

The amount of each ingredient formulated shown in Examples and Comparative Examples below is % by mass with respect to the total amount. The amounts of prototypes 1 to 3 (which comprise degradation products obtained from a DNA salt, from DNA and a nucleoprotein, and from RNA, respectively, by enzymatic degradation treatment) used in Examples are indicated in solid contents.

### 1. Production of deoxyoligonucleotide

DNA derived from salmon milt was subjected to limited degradation with nuclease [e.g., enzyme preparation nuclease "Amano" (manufactured by Amano Enzyme Inc.)] approved as a food additive. The produced deoxymononucleotide and deoxyoligonucleotide were analyzed with an electrophoresis apparatus to determine the optimal conditions.

Specifically, DNA-Na salt powders were added as a raw material to hot water adjusted to approximately 65°C. The solution was stirred and then further heated to 70°C. Nuclease was added in an appropriate amount that fell within a range of 0.05 to 0.25% to the raw material. The solution was reacted for 3 hours. Next, the nuclease was inactivated by heating at 85°C for 10 minutes. Then, the solution was centrifuged. A spray dry method was applied to the supernatant to obtain dried powders (degradation product) containing a deoxyoligonucleotide.

### 2. Analysis of deoxyoligonucleotide

DNA-Na salt powders derived from salmon milt were added as a raw material to hot water adjusted to approximately 65°C. The solution was stirred and then further heated to 70°C. Then, 0.05% enzyme preparation nuclease "Amano" (manufactured by Amano Enzyme Inc.) was added to the raw material. The solution was reacted for 3 hours to obtain a degradation product. Next, the nuclease was inactivated by heating at 85°C for 10 minutes. Then, the degradation product was analyzed by HPLC.

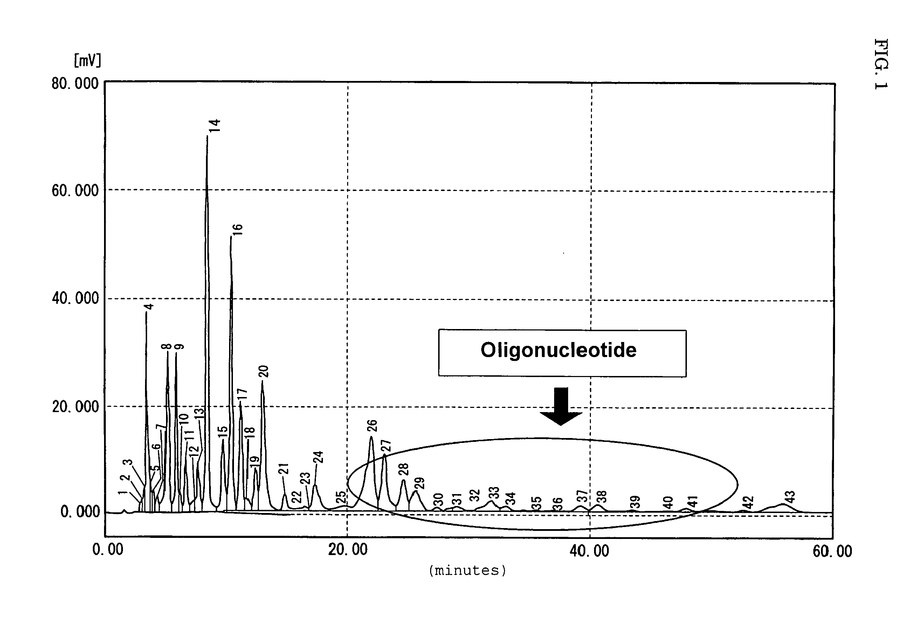

FIG. 1 shows an HPLC (high-performance liquid chromatography) analysis example of the deoxyoligonucleotide in the degradation product. In FIG. 1, a 5'-deoxymononucleotide and a 3'-deoxymononucleotide are eluted before peak 20, and subsequent relatively large peaks, that is, peak 26 and subsequent peaks can be regarded as the absorption of the deoxyoligonucleotide. Moreover, peak 41 and subsequent peaks can be regarded as the absorption of degradation products with a molecular weight exceeding 3000. Therefore, calculation results from the strength of the peaks 26 to 41 demonstrated that 31% deoxyoligonucleotide (molecular weight of 1000 to 3000) fractions with respect to the whole degradation product were contained in this example.

### 3. Production of cosmetics using deoxyoligonucleotide

The deoxyoligonucleotide-containing dried powders (DNA salt degradation product) obtained in the production method was used as a prototype 1. This prototype 1 was used to produce cosmetics of the present invention as described below. Moreover, a cosmetic free of the prototype 1 was produced as a control. The composition of these cosmetics is shown together in Table 1 below.

### [Example 1]

Purified water was added to 95% ethanol. To this solution, polyoxyethylene (25 moles) ether of hydrogenated castor oil, glycerin, and propylene glycol were added and stirred. Then, the prototype 1 was added thereto and dissolved by stirring to obtain a clear liquid cosmetic of Example 1.

### [Comparative Example 1]

Cosmetics of Comparative Example 1 was obtained in the same production method as in Example 1 except that glycerin was used instead of the prototype 1 in the same amount.
[Table 1]

**Table 1: Composition of cosmetics of Example 1 and Comparative Example.**

| Formulated ingredient | Example 1 | Comparative Example 1 |
|---|---|---|
| Prototype 1 (DNA salt degradation Product) | 2.0 | - |
| Concentrated glycerin | 2.0 | 4.0 |
| Polyoxyethylene (25 moles) ether of hydrogenated castor oil | 2.0 | 2.0 |
| Propylene glycol | 5.0 | 5.0 |
| 95% ethyl alcohol | 1.0 | 1.0 |
| Purified water | Remaining | Remaining |

### 4. Evaluation of skin state using skin image analysis system

Each of the cosmetics of Example 1 and Comparative Example 1 was applied in an amount of approximately 1 g once daily after bathing to the left or right half facial skins of subjects in their thirties and in their fifties.

A Robo skin analyzer CS50 system set manufactured by Inforward Inc. was used to evaluate the skin state of the cosmetic-applied portion before start of the test (initial value) and after 4 weeks.

### 4-1. Evaluation of moisture and sebum levels

Moisture and sebum levels were measured using oil/moisture content sensor. The sensor was put on the left and right cheeks. The oil and moisture contents were measured by a measurement method described below, and the measurement results were evaluated according to evaluation criteria described below.

Oil Content (Level of sebum secreted to skin surface)

A rolled oil content was measured on the basis of a refractive index by pressing the sensor portion to the cheeks and evaluated on the basis of an area as follows: the state in which oil covered the whole surface was defined as a "saturated state of oil (100)", and the state in which oil was totally absent was defined as a "zero state of oil (0)".

### · Moisture Content (Moisture level in outer layer of organism)

The capacitance of the skin was measured as an index of a moisture content by pressing the sensor portion to the cheeks and evaluated as follows: the state in which a saline itself was measured was defined as a "saturated state (100)", and the state in which moisture was totally absent was defined as a "zero state (0)".

### 4-2. Evaluation of skinditch density (skin texture)

The state of skinditch density in the left and right cheeks was photographed with a microscope. The photographed images were analyzed and evaluated with the Robo skin analyzer.

The analysis method involves comparing a result of separately processing, for binarization, dark and bright sections in the photographed images (converted to monochrome images) regarded as skinditches and skin hills (crista cutis), respectively, with a regular triangular texture model (submental skin state as reference) of 0.4 mm in side to evaluate the photographed images with the model as 100.

### 4-3. Evaluation of wrinkle

The state of wrinkles under the eyes was evaluated by the analysis of skin images measured with a total facial photographic box.

Such dark sections as to create a significantly steep concentration gradient in the photographed images (converted to monochrome images) were detected as wrinkles under the eyes. The number of wrinkles was counted.

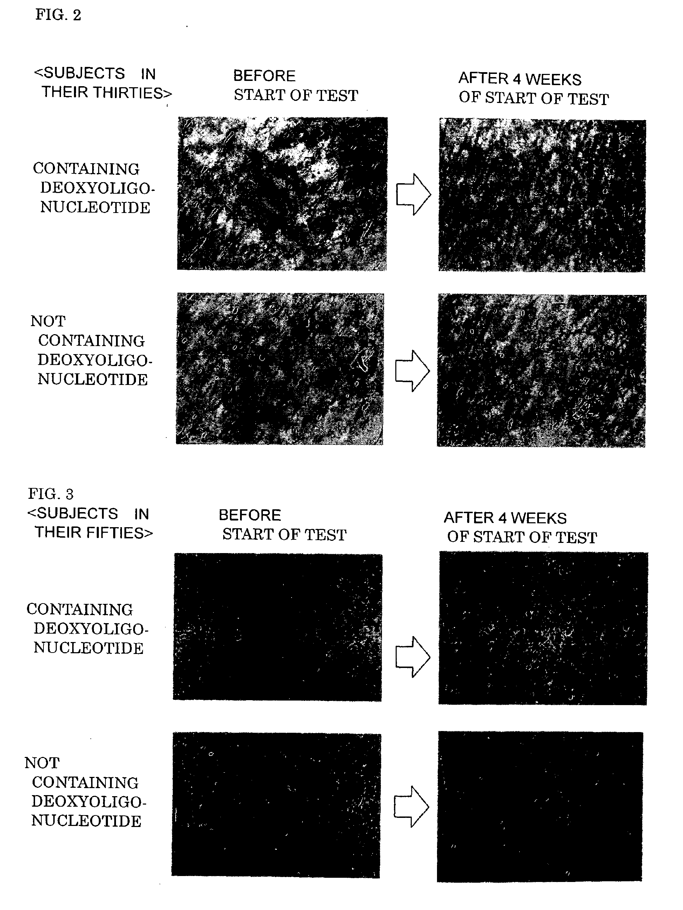

The evaluation of the tests is shown in Tables 2 (subjects: 30's) and 3 (subjects: 50's) below. Changes in the skin state (photographed image) of each subject are shown in FIGS. 2 and 3.

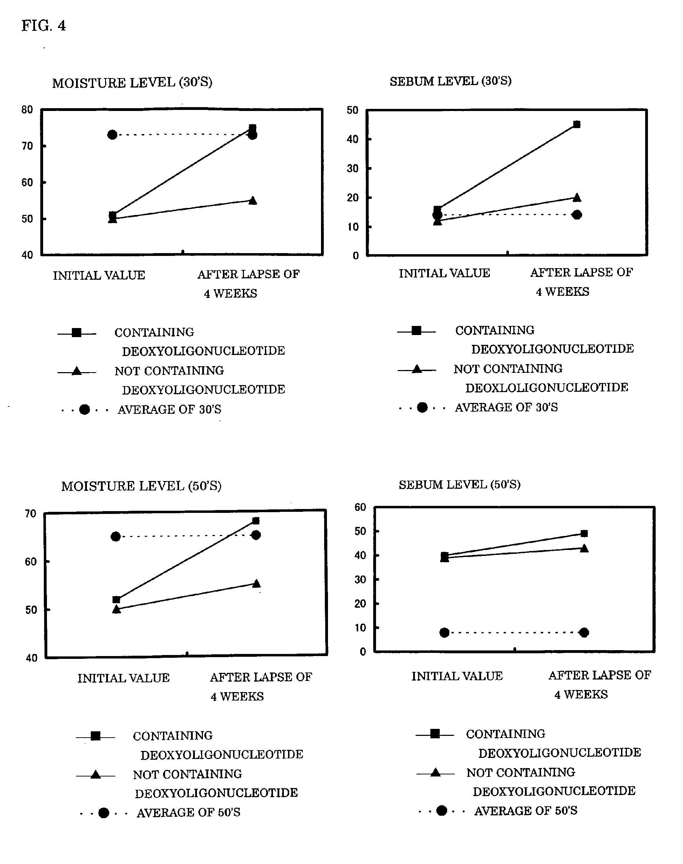

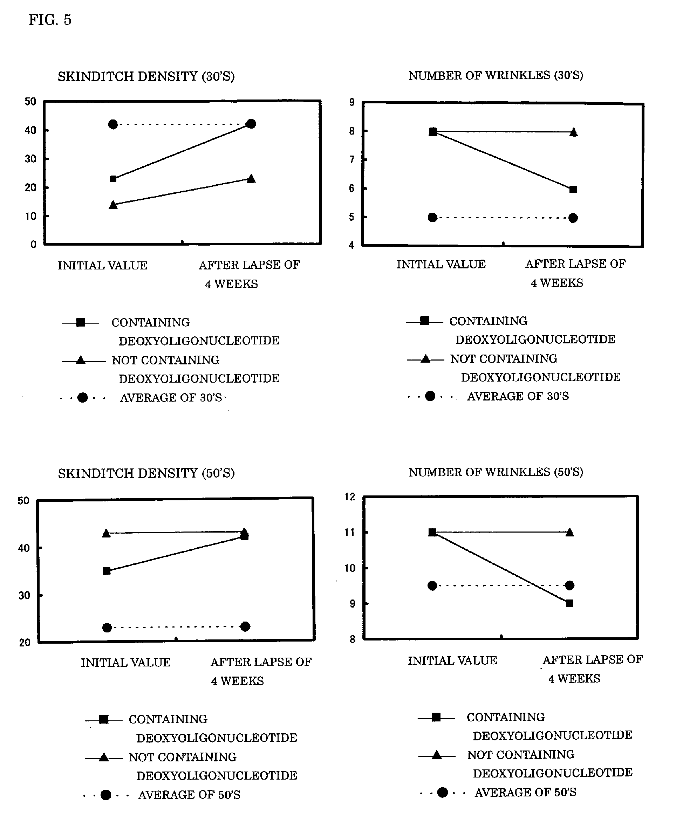

Moreover, diagrams (graphs) illustrating the test results and an average value of each age bracket are shown in FIGS. 4 and 5.
[Table 2]

**Table 2: Evaluation test results of cosmetics of Example 1 and Comparative Example 1 (30's)**

| Evaluation items | | Example 1 | Comparative Example 1 |
|---|---|---|---|
| Moisture level (0-100) | Initial value | 51 | 50 |
| | After lapse of 4 weeks | 75 | 55 |
| | Amount of change (%) | +47 | +10 |
| Sebum level (0-100) | Initial value | 16 | 12 |
| | After lapse of 4 weeks | 45 | 20 |
| | Amount of change (%) | +181 | +67 |
| Skinditch density (0-100) | Initial value | 23 | 14 |
| | After lapse of 4 weeks | 42 | 23 |
| | Amount of change (%) | +83 | +64 |
| The number of wrinkles | Initial value | 8 | 8 |
| | After lapse of 4 weeks | 6 | 8 |
| | Amount of change (%) | -25 | 0 |

[Table 3]

**Table 3: Evaluation test results of cosmetics of Example 1 and Comparative Example 1 (50's)**

| Evaluation items | | Example 1 | Comparative Example 1 |
|---|---|---|---|
| Moisture level (0-100) | Initial value | 52 | 50 |
| | After lapse of 4 weeks Amount of change (%) | 68 | 55 |
| | | +31 | +10 |
| Sebum level (0-100) | Initial value | 40 | 39 |
| | After lapse of 4 weeks | 49 | 43 |
| | Amount of change (%) | +23 | +10 |
| Skinditch density (0-100) | Initial value | 35 | 43 |
| | After lapse of 4 weeks | 42 | 43 |
| | Amount of change (%) | +20 | 0 |
| The number of wrinkles | Initial value | 11 | 11 |
| | After lapse of 4 weeks | 9 | 11 |
| | Amount of change (%) | -18 | 0 |

In the results of Example 1, moisture and sebum levels and skinditch density, as shown in Tables 2 and 3 and FIGS. 4 and 5, were observed to be improved in the subjects both in their thirties and in their fifties. Particularly, the moisture levels (30's and 50's) and the skinditch density (30's) that fell short of the average value before start of the test recovered to the average value after a lapse of 4 weeks. Likewise, the number of wrinkles was decreased to the average value or close to the average value after a lapse of 4 weeks.

From the photographed images shown in FIGS. 2 and 3, skinditch density (texture) could also be confirmed by visual observation to be improved.

On the other hand, Comparative Example 1 had a somewhat improved or similar outcome after a lapse of 4 weeks as compared with before start of the test (initial value) by virtue of the moisturizing effects of the formulated glycerin and the like. However, the results of Comparative Example 1 differed greatly from those of Example 1 in their improving effects.

### 5. Production and analysis of DNA and nucleoprotein degradation products (prototype 2) or RNA degradation product (prototype 3)

A prototype 2 used comprised, as an active ingredient, degradation products obtained from DNA and a nucleoprotein (manufactured by Nissei Bio Co., Ltd.) by enzymatic degradation treatment (comprising a deoxyoligonucleotide, a deoxymononucleotide, and an oligopeptide). A prototype 3 used comprised, as an active ingredient, a degradation product obtained from RNA (manufactured by Nissei Bio Co., Ltd.) by enzymatic degradation treatment (comprising an oligonucleotide and a mononucleotide).

Production methods and analysis results of the prototypes 2 and 3 are shown below.

### 5-1. Production method and analysis of prototype 2 (DNA and nucleoprotein degradation products)

The prototype 2 was produced by the same production method as in the prototype 1. Specifically, degradation products obtained from DNA and from a nucleoprotein by enzymatic degradation treatment for reducing the molecular weight were mixed in equal amounts. Details of a production method of each degradation product are given below.

### <DNA degradation product>

### [Production method]

Production was performed in the same way as in the paragraph "1. Production of deoxyoligonucleotide" to obtain a DNA degradation product.

### [Structure and composition]

Analysis conducted in the same way as in the paragraph "2. Analysis of deoxyoligonucleotide" demonstrated that 31% deoxyoligonucleotide (molecular weight of 1000 to 3000) fractions with respect to the whole degradation product were contained in this example.

### <Nucleoprotein degradation product>

### [Production method]

A nucleoprotein (manufactured by Nissei Bio Co., Ltd.) derived from salmon milt was added as a raw material to water. The solution was heated at 50°C and stirred. Then, 0.065% enzyme preparation protease "PTN" (manufactured by Novozymes Japan Ltd.) was added to the raw material. The solution was reacted for 4 hours. The reaction solution was further heated at 70°C and stirred. Subsequently, 0.1% enzyme preparation nuclease "Amano" (manufactured by Amano Enzyme Inc.) was added thereto. The solution was reacted for 3 hours. Next, the nuclease was inactivated by heating at 85°C for 10 minutes. Then, the solution was centrifuged. A spray dry method was applied to the supernatant to obtain dried powders (degradation product) containing a deoxyoligonucleotide.

### [Structure and composition]

The obtained degradation product was analyzed by HPLC.

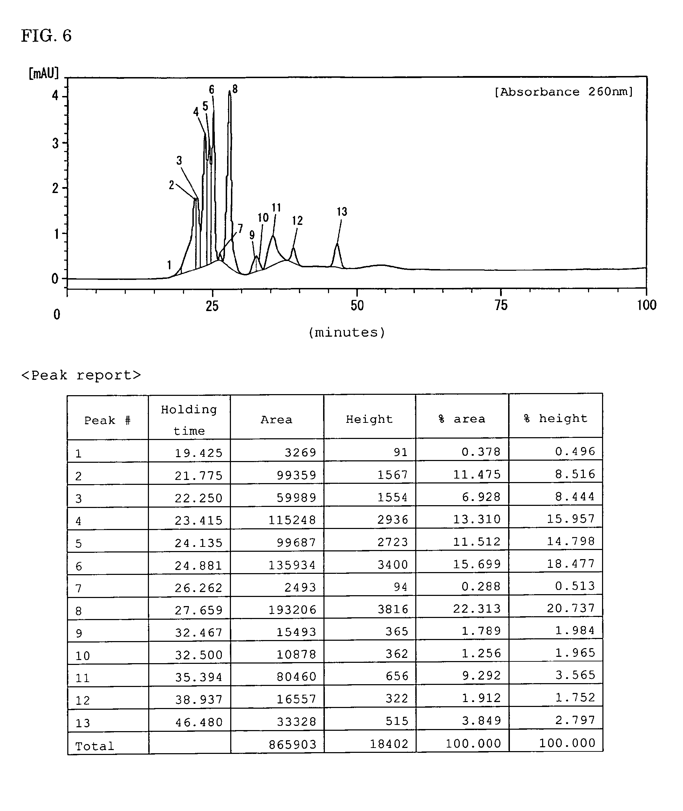

FIG. 6 shows an HPLC (high-performance liquid chromatography) analysis example of the deoxyoligonucleotide in the degradation product. In FIG. 6, peaks (peaks 1 to 4) within the retention time from 19 minutes to 24 minutes can be regarded as the absorption of the deoxyoligonucleotide. Therefore, calculation results from the strength of the peaks 1 to 4 demonstrated that 33.4% deoxyoligonucleotide (molecular weight of 1000 to 3000) fractions with respect to the whole degradation product were contained in this example. 5-2. Production method and analysis of prototype 3 (RNA degradation product)

The prototype 3 was produced by the same production method as in the prototype 1. Specifically, the prototype 3 is a degradation product obtained from RNA instead of the raw material DNA by enzymatic degradation treatment for reducing the molecular weight. Details thereof are given below.

### <RNA degradation product>

### [Production method]

RNA (manufactured by Nissei Bio Co., Ltd.) derived from yeast was added as a raw material to hot water adjusted to approximately 70°C. The solution was stirred and then further heated to 70°C. Then, 0.05% enzyme preparation nuclease "Amano" (manufactured by Amano Enzyme Inc.) was added to the raw material. The solution was reacted for 3 hours. Next, the nuclease was inactivated by heating at 85°C for 10 minutes. Then, the solution was centrifuged. A spray dry method was applied to the supernatant to obtain dried powders (degradation product) containing an oligonucleotide.

### [Structure and composition]

The obtained degradation product was analyzed by HPLC.

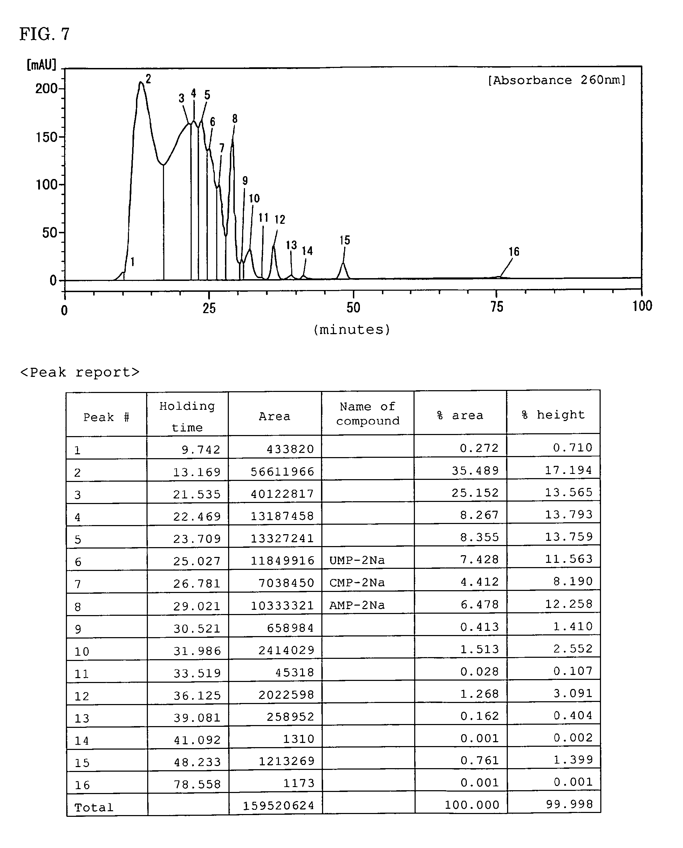

FIG. 7 shows an HPLC (high-performance liquid chromatography) analysis example of the oligonucleotide in the degradation product. In FIG. 7, peaks (peaks 2 to 5) within the retention time from 13 minutes to 24 minutes can be regarded as the absorption of the oligonucleotide. Therefore, calculation results from the strength of the peaks 2 to 5 demonstrated that 41.1% oligonucleotide (molecular weight of 1000 to 3000) fractions with respect to the whole degradation product were contained in this example.

### 6. Production of a variety of cosmetics

The prototype 2 used comprised, as an active ingredient, degradation products obtained from DNA and a nucleoprotein (manufactured by Nissei Bio Co., Ltd.) by enzymatic degradation treatment (comprising a deoxyoligonucleotide, a deoxymononucleotide, and an oligopeptide). The prototype 3 used comprised, as an active ingredient, a degradation product obtained from RNA (manufactured by Nissei Bio Co., Ltd.) by enzymatic degradation treatment (comprising an oligonucleotide and a mononucleotide).

The obtained prototypes 2 and 3 were used to produce cosmetics of the present invention as described below. Moreover, cosmetics of Comparative Examples free of the prototypes 2 and 3 were produced as controls. The composition of these cosmetics is shown together in Tables 4 to 7 below.

Manufacturers from which the products used in the cosmetics shown below were obtained are shown in parentheses.

### 6-1. Cosmetic in gel form

### [Example 2 (Examples 2-1 to 2-3)]

Pemulen TR-1 (Nikko Chemicals Co., Ltd.) was gradually added to purified water with stirring and sufficiently dispersed. Subsequently, Ultrez-10 (Nikko Chemicals Co., Ltd.) was dispersed therein by stirring. Then, concentrated glycerin was added thereto to prepare a gel base. On the other hand, Lecinol WS-50 (Nikko Chemicals Co., Ltd.), jojoba oil, squalane, LIALCARB SR-1000 (Nikko Chemicals Co., Ltd.), AMITER MA-HD (Nihon-Emulsion Co., Ltd.), and rose hip oil were heat-melted and mixed to prepare an oily ingredient. On the other hand, Ceralipid W-2 (Nikko Chemicals Co., Ltd.), 1,3-butylene glycol (BG), dipropylene glycol (DPG), and 2-phenoxy ethanol were added. The solution was heated and mixed by stirring (300 rpm, 80°C, 5-minute retention). Purified water was added thereto. After sufficient stirring, this solution was combined with the oily ingredient and sufficiently mixed to prepare an undiluted solution of the formulated ingredients.

The gel base and the undiluted solution of the formulated ingredients were mixed. Ethanol was added thereto. Then, the solution was neutralized by the addition of 18% sodium hydroxide and mixed by stirring. Each of the prototype 2, the prototype 3, and a mixture of the prototypes 2 and 3 in equal amounts (hereinafter, referred to as a prototype 4) mixed with purified water was dissolved in this solution to prepare cosmetics in a gel form of Examples 2-1 to 2-3.

### [Comparative Example 2]

A cosmetic in a gel form of Comparative Example 2 was prepared in the same way as in Examples 2-1 to 2-3 except that additional 2% by mass of concentrated glycerin was used instead of the prototypes 2 and 3 (7% by mass in total of concentrated glycerin was added).

### 6-2. Cosmetic in milky liquid form

### [Example 3 (Examples 3-1 to 3-3)]

Concentrated glycerin was added to purified water and heated to 70°C to prepare an aqueous phase. On the other hand, monooleate and glycerol monostearate were added to cetyl alcohol, beeswax, vaseline, squalane, and dimethylpolysiloxane and heated to 70°C. This solution was added to the aqueous phase prepared in advance to perform preliminary emulsification. A quince seed extract and ethanol were further added thereto and stirred to make the emulsified particles uniform with a homomixer.

At the point in time when this solution in a milky liquid form was brought to 40°C, each of the prototype 2, the prototype 3, and the mixture of the prototypes 2 and 3 in equal amounts (prototype 4) mixed with purified water was dissolved in this solution to prepare cosmetics in a milky liquid form of Examples 3-1 to 3-3.

### [Comparative Example 3]

A cosmetic in a milky liquid form of Comparative Example 3 was prepared in the same way as in Examples 3-1 to 3-3 except that additional 2% by mass of concentrated glycerin was used instead of the prototypes 2 and 3 (10% by mass in total of concentrated glycerin was added).

### 6-3. Cosmetic in lotion form

### [Example 4 (Examples 4-1 to 4-3)]

In purified water, 1,3-butylene glycol, concentrated glycerin, a buffer, and an anti-browning agent were dissolved at room temperature. On the other hand, oleyl alcohol, sorbitan monolaurate, and each of the prototype 2, the prototype 3, and the mixture of the prototypes 2 and 3 in equal amounts (prototype 4) mixed with purified water were separately dissolved in ethanol. These solutions were mixed by stirring to prepare cosmetics in a lotion form of Examples 4-1 to 4-3.

### [Comparative Example 4]

A cosmetic in a lotion form of Comparative Example 4 was prepared in the same way as in Examples 4-1 to 4-3 except that additional 2% by mass of concentrated glycerin was used instead of the prototypes 2 and 3 (6% by mass in total of concentrated glycerin was added).

### 6-4. Cosmetic in cream form

### [Example 5 (Examples 5-1 to 5-3)]

In purified water, 7.,3-butyl.ene glycol, pentylene glycol, concentrated glycerin, and polyquaternium were sequentially dissolved and heated to 80°C to prepare an aqueous phase. On the other hand, polyglyceryl stearate, stearyl alcohol, behenyl alcohol, batyl alcohol, cetyl palmitate, glycerin stearate, Crodalan SWL (Croda Japan KK), isopropyl palmitate, squalane, octyldodecyl myristate, macadamia nut oil, trioctanoin, and dimethicone were mixed. The mixture was heated at 85°C and stirred to prepare an oil phase. The oil phase was poured to the aqueous phase and stirred (300 rpm). The mixture was emulsified with a homomixer for high viscosity (4000 rpm).

At the point in time when this emulsified substance was brought to 40°C, sodium hydroxide, sodium citrate, and each of the prototype 2, the prototype 3, and the mixture of the prototypes 2 and 3 in equal amounts (prototype 4) mixed with purified water were separately dissolved in purified water and mixed with the emulsified substance by stirring to prepare cosmetics in a cream form of Examples 5-1 to 5-3.

### [Comparative Example 5]

A cosmetic in a cream form of Comparative Example 5 was prepared in the same way as in Examples 5-1 to 5-3 except that additional 2% by mass of concentrated glycerin was used instead of the prototypes 2 and 3 (7% by mass in total of concentrated glycerin was added).

### 7. Performance test of cosmetics (gel, milky lotion, lotion, and cream)

The cosmetics of Examples 2 to 5 and Comparative Examples 2 to 5 were evaluated as described below. The results are shown together in Tables 4 to 7.

### 7-1. State of skinditch density (texture)

Each of the cosmetics of Examples 2 to 5 and Comparative Examples 2 to 5 was applied in an amount of approximately 1 g once daily after bathing to the left or right half facial skins of female subjects in late middle age.

The Robo skin analyzer CS50 system set manufactured by Inforward Inc. was used to evaluate skinditch density in the skin of the cosmetic-applied portion after 4 weeks as described above. The test results were assessed according to the following assessment criteria:
++: the numeric value of skinditch density in the cosmetic-applied portion was increased by 10% or more relative to Comparative Example (significantly effective);
+: the numeric value of skinditch density in the cosmetic-applied portion was increased by 5 to 10% relative to Comparative Example (effective);
±: the numeric value of skinditch density in the cosmetic-applied portion was increased by 0 to 5% relative to Comparative Example (slightly effective); and
-: the numeric value of skinditch density in the cosmetic-applied portion was equal to or lower than that of Comparative Example (ineffective).

### 7-2. Skin roughness improvement test

Each of the cosmetics of Examples 2 to 5 and Comparative Examples 2 to 5 was applied in an amount of approximately 1 g once daily after bathing to different positions of the left and right legs of 10 female subjects in late middle age having skin roughness in the legs.

The test results were assessed according to assessment criteria below from the viewpoint of skin exfoliation and moisture states on the basis of the skin state of the cosmetic-applied portion before start of the test and after 1 month.

### 7-2-1. Assessment criteria of skin exfoliation and determination of effectiveness

A skin exfoliation state before start of the test and after 1 month was assessed according to the following assessment criteria:

0: no exfoliation, 1: mild exfoliation, 2: moderate exfoliation, and 3: heavy exfoliation.

According to these assessment criteria, those assessed as being improved after 1 month by one stage from the state before start of the test were determined as "slightly effective"; those assessed as being improved after 1 month by two stages from the state before start of the test were determined as "effective"; and those assessed as being equal to or worse than the state before start of the test were determined as "ineffective".

### 7-2-2. Assessment criteria of moisture state

A skin moisture state (moisture retention properties) after 1 month was measured with a moisture meter and assessed according to the following criteria:

### <Assessment criteria of moisture state>

++: the moisture retention properties of the cosmetic-applied portion were increased by 5% or more relative to the state before start of the test (significantly effective);
+: the moisture retention properties of the cosmetic-applied portion were increased by 2 to 5% relative to the state before start of the test (effective) ;
±: the moisture retention properties of the cosmetic-applied portion were increased by 0 to 2% relative to the state before start of the test (slightly effective); and
-: the moisture retention properties of the cosmetic-applied portion were equal to or lower than the state before start of the test (ineffective).

### 7-3. Evaluation test by trial use

The evaluation test of the cosmetics of Examples 2 to 5 and Comparative Examples 2 to 5 was conducted on female subjects in late middle age by a single blind test. In this test, each cosmetic involved 10 subjects. Evaluation was conducted by questionnaires about two items, "smoothness" and "fineness of texture", to which the subjects replied before use and after 1-month use.

From the obtained replies, the test results were assessed according to the following assessment criteria:
++: improved as compared with the state before use (effective);
+: slightly improved as compared with the state before use (slightly effective); and
±: equal to or lower than the state before use (ineffective).

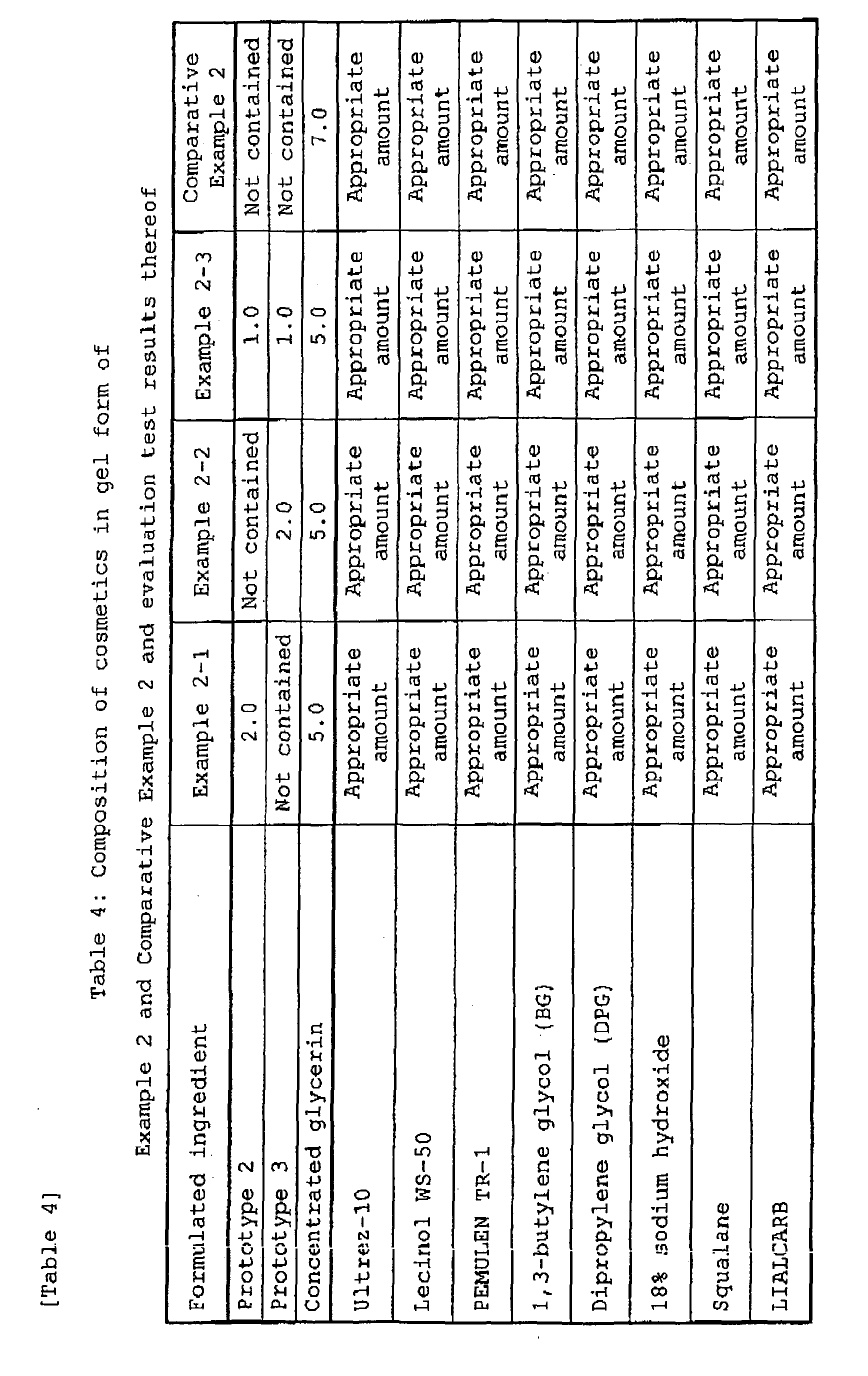

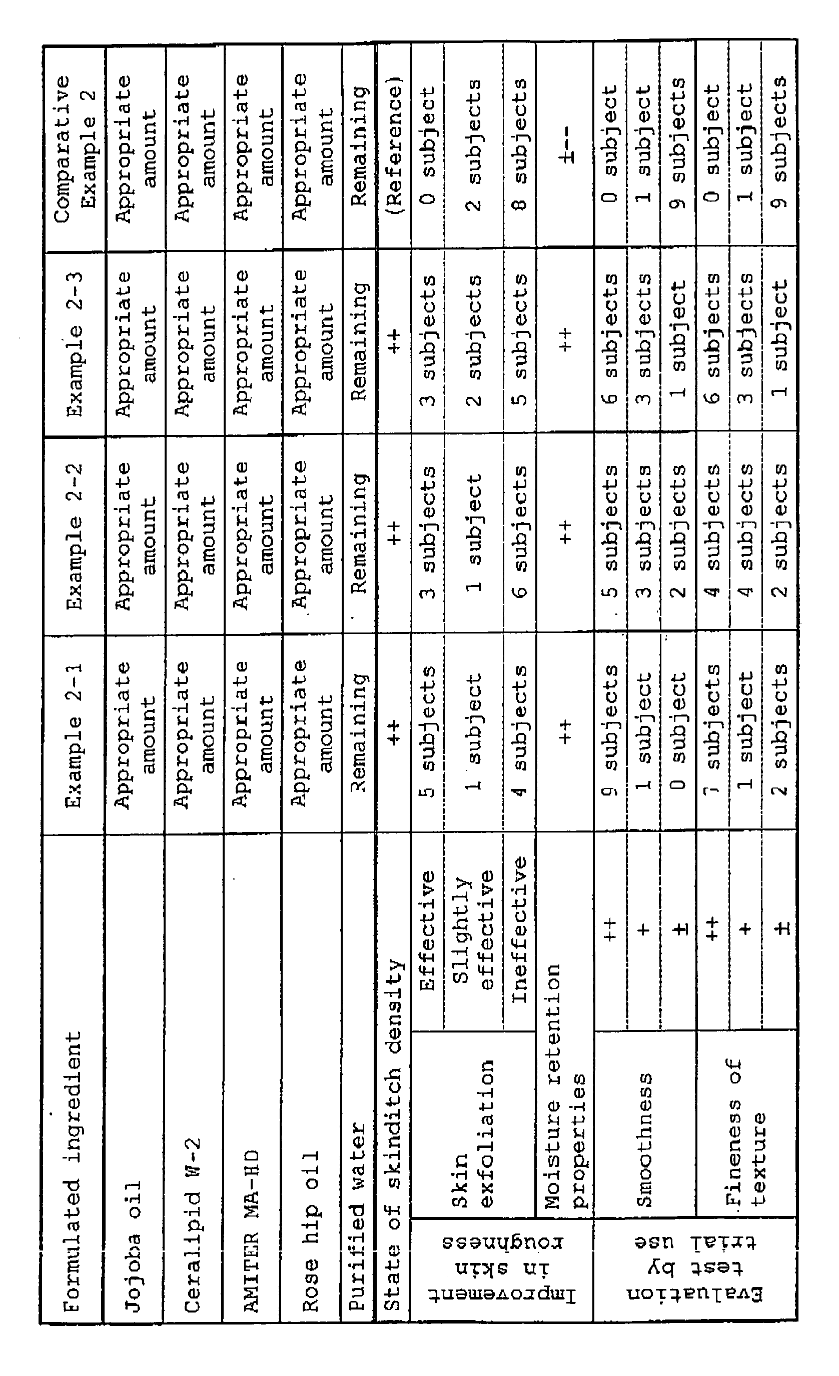

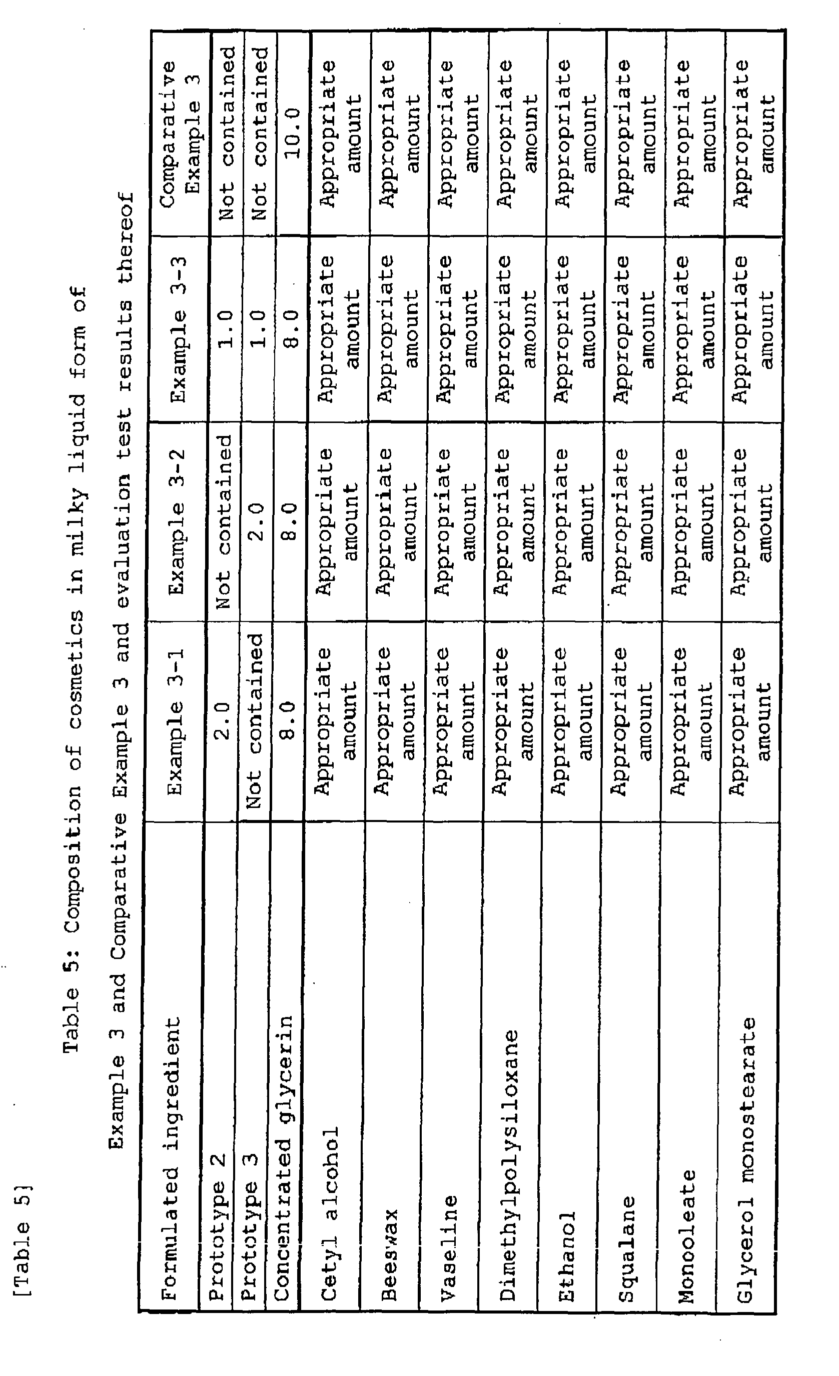

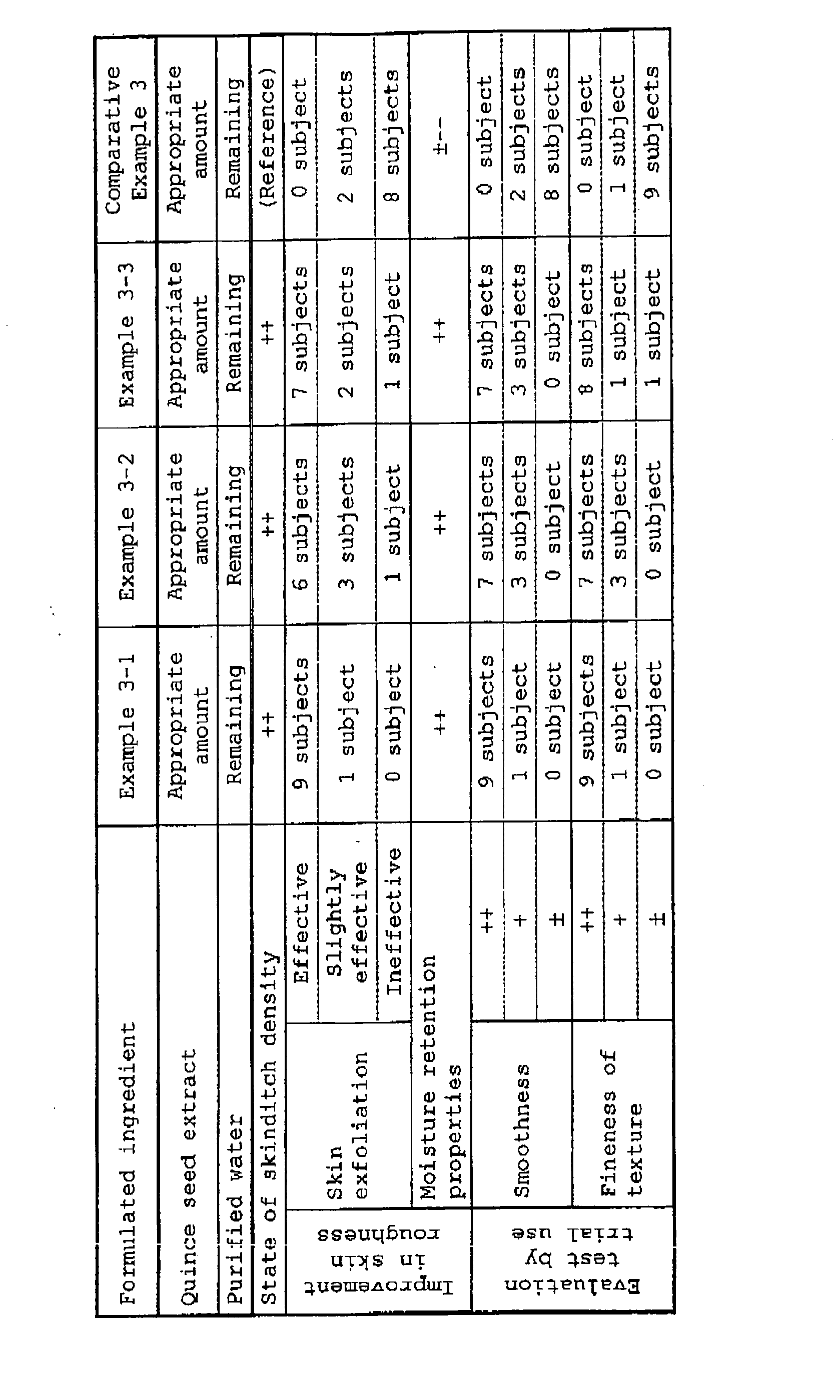

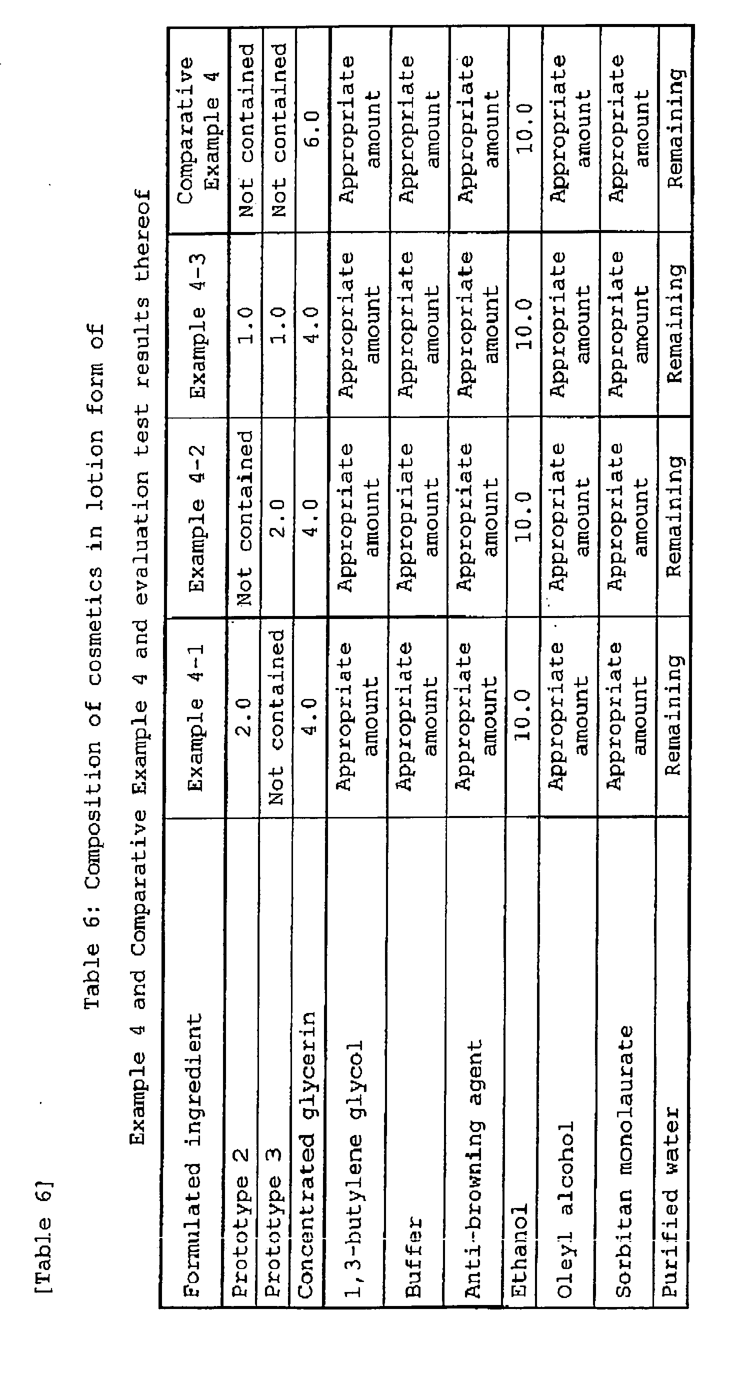

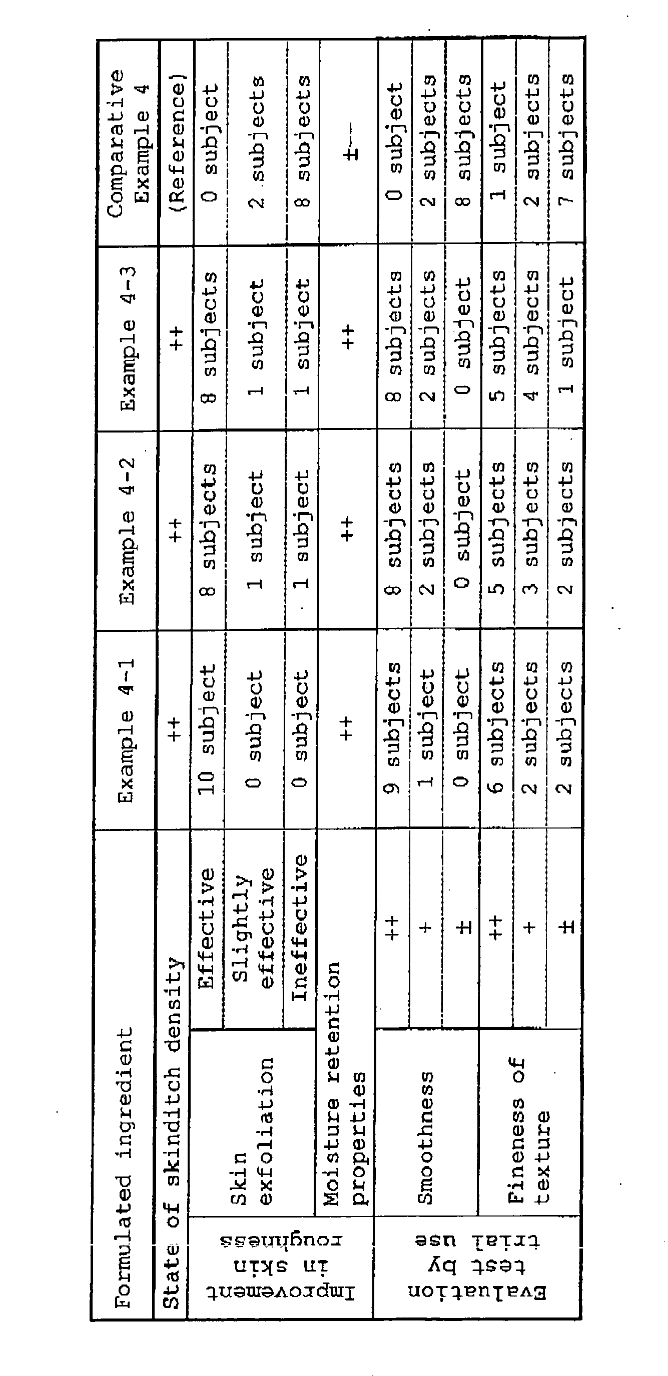

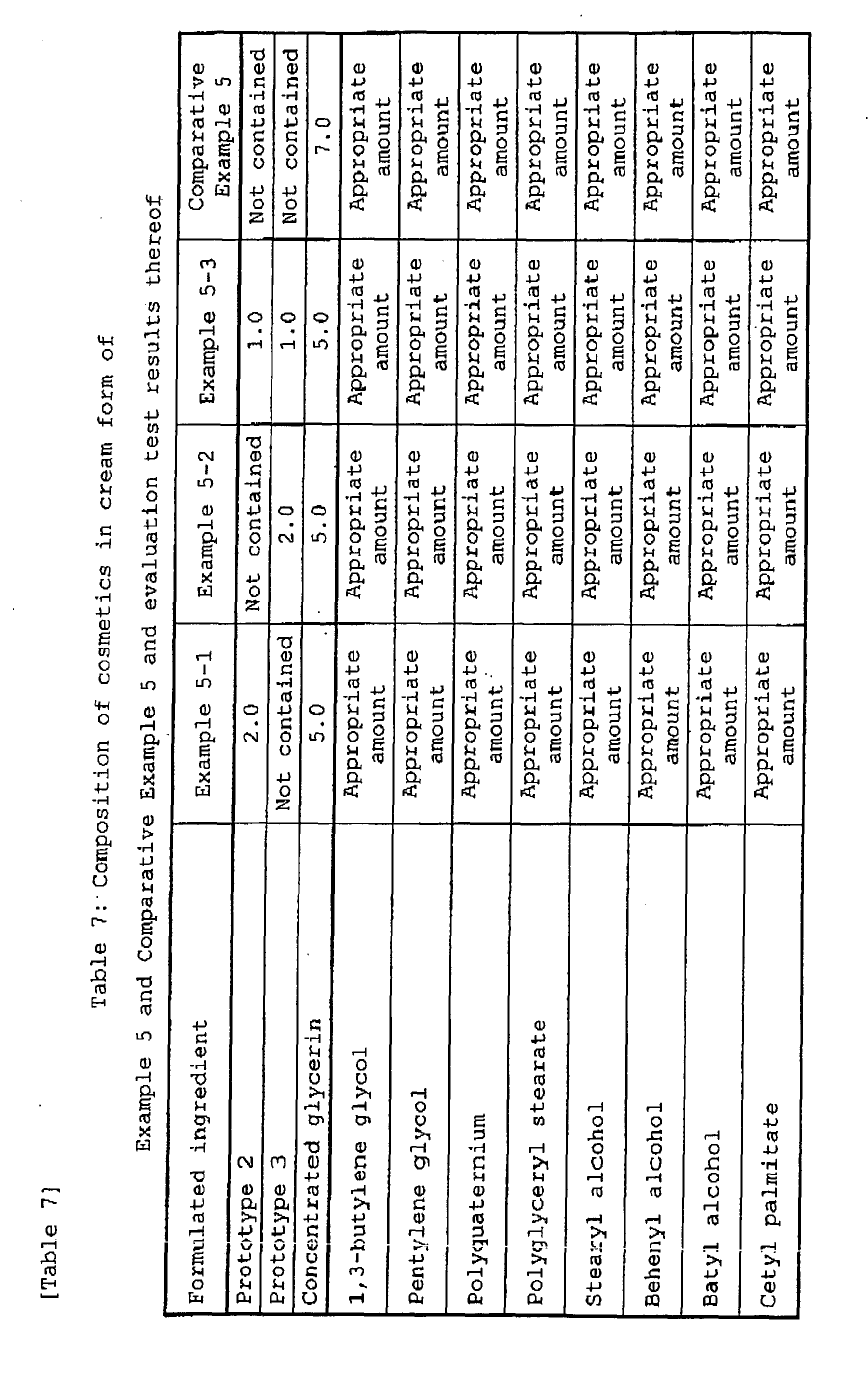

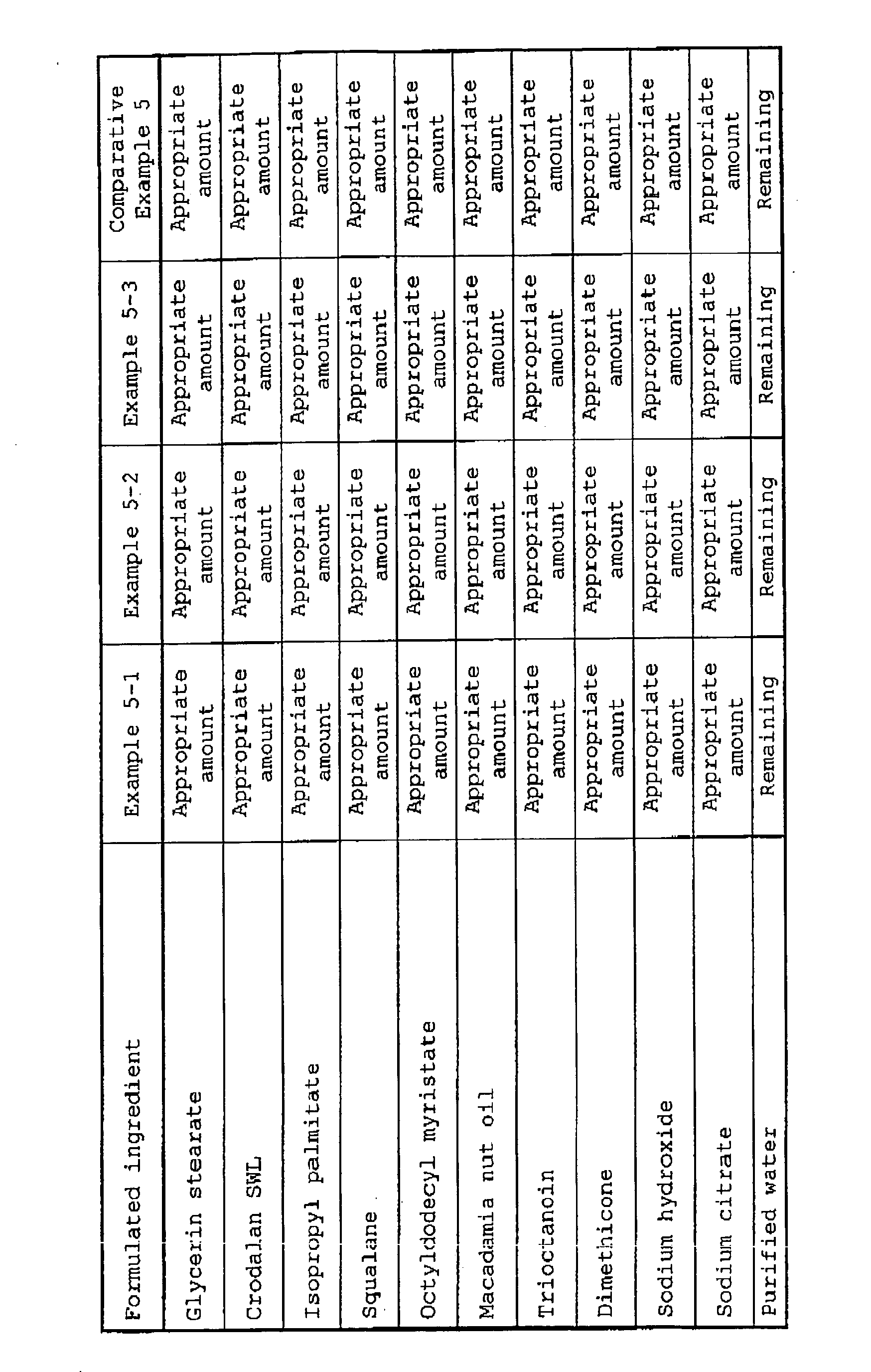

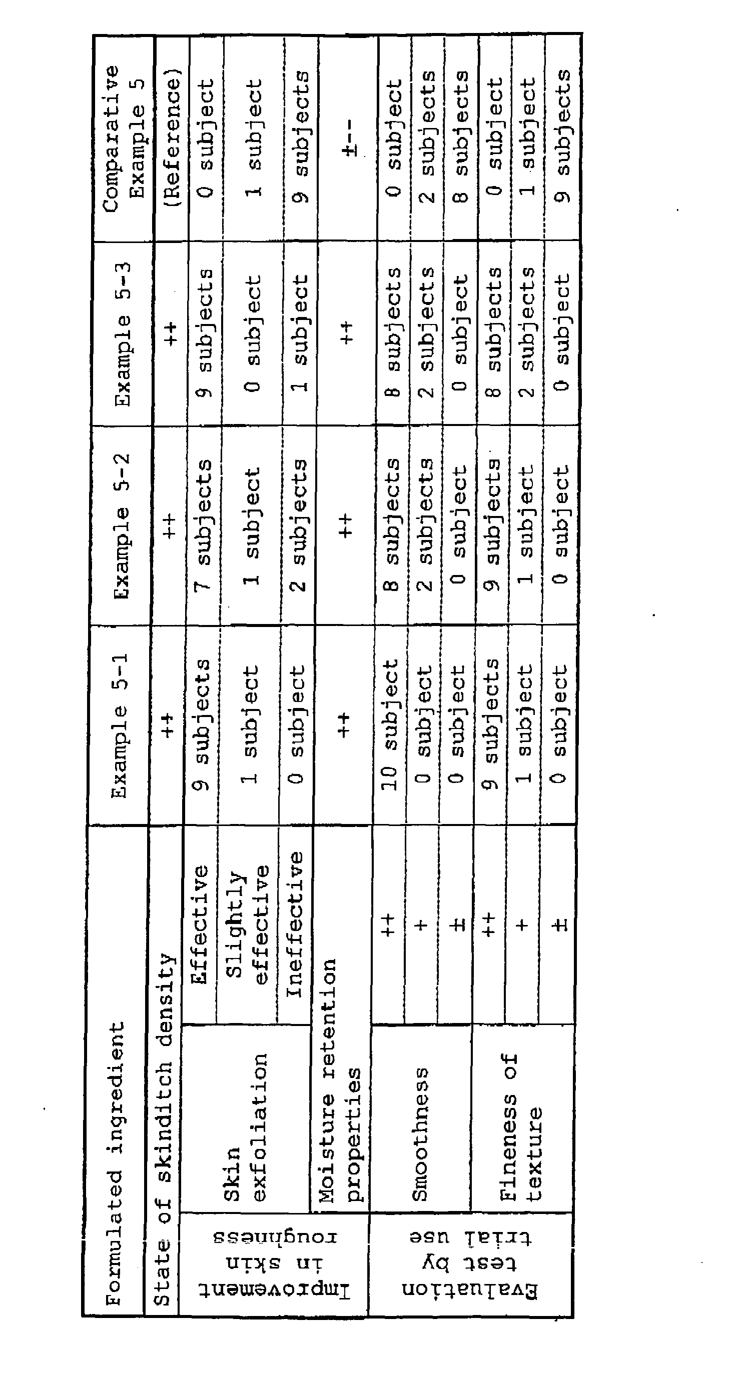

As shown in Tables 4 to 7, the cosmetics of Examples 2 to 5 comprising the prototype 2, 3, or 4 (mixture of the prototypes 2 and 3 in equal amounts) exhibited significant improvement in the state of skinditch density as compared with the cosmetics of Comparative Examples 2 to 5 free from the prototypes 2 to 4.

Moreover, the test results of the cosmetics of Examples 2 to 5 also showed almost significant effectiveness in the skin roughness improvement test and significant improving effects in the evaluation test by trial use.

On the other hand, the improving effects of the cosmetics of Comparative Examples 2 to 5 were hardly observed both in the skin roughness improvement test and in the evaluation test by trial use, demonstrating the effectiveness of the cosmetics comprising the prototypes 2 to 4.

In these Examples 1 to 5, the prototypes 1 (DNA salt degradation product), 2 (DNA and nucleoprotein degradation products), 3 (RNA degradation product), and 4 (mixture of the prototypes 2 and 3) were used as active ingredients. However, even when a deoxyoligonucleotide, a deoxymononucleotide, an oligopeptide, an oligonucleotide, and a mononucleotide separated and purified from the prototypes 1 to 4 were used alone or in combination instead of the prototypes 1 to 4, the same effects as produced by the prototypes 1 to 4 were observed.

Specifically, from the present Examples, a preparation comprising a deoxyoligonucleotide or the like (at least one of a deoxyoligonucleotide, a deoxymononucleotide, an oligopeptide, an oligonucleotide, and a mononucleotide) was confirmed to have the effect of improving skin states (moisture and sebum levels, skinditch density, and wrinkles). Particularly, the test results showing improved skinditch density can be said to support the idea that the deoxyoligonucleotide or the like has a cellular regeneration or activation ability.

Hereinafter, cosmetic formula examples using the prototypes 1 to 4 will be shown as Examples 6 to 8. The "prototype(s)" described herein refers to any ingredient selected from the prototypes 1 to 3 used alone and the mixture of the prototypes 2 and 3 in equal amounts (prototype 4).

In all of Examples below, the cosmetics were confirmed to improve skin moisture and sebum levels and the state of skinditch density and have the effect of reducing wrinkles. These effects could be confirmed by trial use. The cosmetics were also confirmed to have the effects of improving skin smoothness and fineness of texture.

### Example 6: Cold cream

[Table 8]

**Table 8 Formula of cold cream**

| Formulated ingredient | Amount of formulated (% by mass) |
|---|---|
| Solid paraffin | 5.0 |
| Beeswax | 10.0 |
| Vaseline | 15.0 |
| Liquid paraffin | 41.0 |
| Glycerin monostearate | 2.0 |
| Polyoxyethylene (20 moles) sorbitan monolaurate | 2.0 |
| Prototype (s) | 1.0 |
| Soap powder | 0.1 |
| Perfume | Appropriate amount |
| Preservative | Appropriate amount |
| Antioxidant | Appropriate amount |
| Purified water | Remaining |

### <Production method>

The prototype(s) and soap powders were added to purified water and heat-melted. This solution was kept at 70°C (aqueous phase). The other ingredients were mixed and heat-melted. This solution was kept at 70°C (oil phase). The oil phase was gradually added to the aqueous phase with stirring. After the completion of the addition, the mixture was uniformly emulsified with a homomixer. After emulsification, the emulsified product was cooled to 30°C with well stirring to prepare a cold cream.

### Example 7: Clay pack

[Table 9]

**Table 9 Formula of clay pack**

| Formulated ingredient | Amount of formulated (% by mass) |
|---|---|
| Prototype(s) | 1.0 |
| Kaolin | 25.0 |
| Ethanol | 5.0 |
| 1,3-butylene glycol | 10.0 |
| Glycerin | 5.0 |
| PEG-20 cetyl glucose sesquistearate | 4.0 |
| Xanthan gum ' | 0.1 |
| Preservative | Appropriate amount |
| Purified water | Remaining |

### <Production method>

1,3-butylene glycol, glycerin, PEG-20 cetyl glucose sesquistearate, xanthan gum, a preservative, and purified water were mixed by stirring to prepare an entirely uniform solution.

The prototype(s) and kaolin were added to this solution with stirring with a homomixer to prepare an entirely uniform solution. Ethanol was further added to the solution and mixed by stirring to prepare a clay pack.

### Example 8: Foundation

[Table 10]

**Table 10: Formula of foundation**

| Formulated ingredient | Amount of formulated (% by mass) |
|---|---|
| Prototype(s) | 2.0 |
| Glycerin oleate | Appropriate amount |
| Triglyceryl diisostearate | Appropriate amount |
| Cyclohexane dioctyl | Appropriate amount |
| Cetyl alcohol | Appropriate amount |
| Microcrystalline wax | Appropriate amount |
| Liquid paraffin | Appropriate amount |
| Trimethylsiloxysilicic acid solution | Appropriate amount |
| Talc | Appropriate amount |
| Iron oxide (pigment) | Appropriate amount |
| Glycerin | Appropriate amount |
| Magnesium sulfate | Appropriate amount |
| Germicide | Appropriate amount |
| Perfume | Appropriate amount |
| Purified water | Remaining |

### <Production method>

Glycerin oleate, triglyceryl diisostearate, cyclohexane dioctyl, cetyl alcohol, microcrystalline wax, liquid paraffin, a trimethylsiloxysilicic acid solution, talc, and iron oxide were heat-melted and mixed (A). Glycerin, magnesium sulfate, and purified water were mixed and heated. The component (A) was added to the mixture and then cooled. The prototype(s) dissolved in purified water were added thereto. Next, a germicide and a perfume were added thereto to prepare a base foundation.

## Claims

1. Compounding ingredients for cosmetics for improving skinditch density, comprising:
a degradation product comprising 20 to 50% fractions with a molecular weight of 1000 to 3000 obtained from a nucleoprotein and/or DNA by enzymatic degradation or hydrolysis treatment for reducing the molecular weight, or a deoxyoligonucleotide, deoxymononucleotide, or oligopeptide separated from the degradation product;
a degradation product comprising 20 to 50% fractions with a molecular weight of 1000 to 3000 obtained from RNA by enzymatic degradation or hydrolysis treatment for reducing the molecular weight, or an oligonucleotide or mononucleotide separated from the degradation product; or
a mixture of at least two members selected from the group consisting of the nucleoprotein and/or DNA degradation product(s), the RNA degradation product, the deoxyoligonucleotide, the deoxymononucleotide, the oligopeptide, the oligonucleotide, and the mononucleotide.

2. The compounding ingredients for cosmetics according to claim 1, wherein the nucleoprotein and the DNA are obtained from milt of fishes such as salmons, trouts, herrings, and cods.

3. The compounding ingredients for cosmetics according to claim 1, wherein the RNA is obtained from yeast selected from the group consisting of beer yeast, torula yeast, milk yeast, and baker's yeast.

4. Cosmetics for improving skinditch density, comprising compounding ingredients for cosmetics according to any of claims 1 to 3.
